# FASCICULE DE BREVET EUROPEEN

(11) **EP 3 310 874 B1**
(45) Date de publication et mention de la délivrance du brevet: **16.12.2020**
(21) Numéro de dépôt: 15745234.3
(22) Date de dépôt: 17.06.2015
(51) Int. Cl.: G01N 33/28, C09K 8/528, E21B 37/06, E21B 47/10, G01N 21/77, G01N 21/64, G01N 31/22, G01N 33/543, G01N 33/58

(54) **METHODE ET SOLUTION DE DOSAGE D'INHIBITEURS DANS UN FLUIDE PETROLIER CONTENANT DE L'EAU**
VERFAHREN UND LÖSUNG ZUR BESTIMMUNG DER MENGE AN INHIBITOREN IN ERDÖLFLÜSSIGKEITEN, DIE WASSER ENTHALTEN
METHOD AND SOLUTION FOR DETERMINING THE AMOUNT OF INHIBITORS IN PETROLEUM FLUIDS COMPRISING WATER

(43) Date de publication de la demande: 25.04.2018
(73) Titulaire: TOTAL SE, 92400 Courbevoie (FR); Université Claude Bernard Lyon I, 69100 Villeurbanne (FR); Centre National de la Recherche Scientifique (CNRS), 75016 Paris (FR)
(72) Inventeur: HURTEVENT, Christian, F-46310 Saint Chamarand (FR); BARAKA-LOKMANE, Salima, F-64000 Pau (FR); TILLEMENT, Olivier, F-69270 Fontaines Saint-Martin (FR); MARAIS, Arthur, F-56650 Lochrist (FR); MARTINI, Mattéo, F-69006 Lyon (FR); OULD-METIDJI, Mahmoud, F-69006 Lyon (FR); VASQUEZ VELADO, Francisco, F-69007 Lyon (FR); LEPOIVRE, Florian, F-69009 Lyon (FR)
(74) Mandataire: Plasseraud IP
(86) Numéro de dépôt international: PCT/FR2015/051608
(87) Numéro de publication internationale: WO 2016/203119

(56) Documents cités:
- WO-A1-2015/075300
- WO-A1-2015/075309
- WO-A1-2015/092310
- US-A1- 2004 082 768
- Thomas Brichart ET AL: "IPTC-17933-MS The Use of Fluorescent Tracers for Inhibitor Concentration Monitoring Useful for Scale Inhibitor Squeeze Evaluation", , 10 décembre 2014 (2014-12-10), XP055257208, Extrait de l'Internet: URL:https://www.onepetro.org/conferences/S PE/15OCS [extrait le 2016-03-10]
- James Johnstone ET AL: "SPE-169797-MS Novel Method for Real-Time Monitoring of Scale Control Products at the Site of Use", , 14 mai 2014 (2014-05-14), pages 14-15, XP055257206, Extrait de l'Internet: URL:https://www.onepetro.org/conferences/S PE/14OSS [extrait le 2016-03-10]

## Description

### DOMAINE TECHNIQUE

La présente invention a pour objet une méthode de détection et quantification d'additifs dans un fluide aqueux complexe, et notamment une méthode de détection d'un inhibiteur de dépôts minéraux ou de corrosion injecté dans un puits de gaz ou de pétrole. Elle a également pour objet une solution révélatrice comprenant un lanthanide et un agent chélateur pour la détection desdits additifs ou inhibiteurs.

### ARRIERE-PLAN DE L'INVENTION

Lors de l'exploitation de champs pétroliers ou gaziers, la récupération de gaz ou de pétrole peut être améliorée en injectant de l'eau dans le gisement, via un puits d'injection, de manière à pousser le pétrole du gisement hors du sous-sol, par au moins un autre puits appelé puits de production. L'interaction de l'eau injectée avec le réservoir contenant le gaz, le pétrole et des eaux souterraines peut résulter en la formation d'espèces chimiques susceptibles d'entraîner des défauts de fonctionnement des installations. Plus particulièrement, des dépôts de sulfate de baryum, de carbonate de calcium ou de sulfures de zinc et de plomb, par exemple, sont notamment susceptibles de se former dans des conditions d'exploitation à haute pression et haute température ou lors de la mise en contact de la saumure injectée pour extraire le pétrole ou le gaz avec le sulfure d'hydrogène ou les ions contenus dans le réservoir. La production d'eau de réservoir ou d'aquifère simultanément avec le pétrole ou le gaz peut entrainer les mêmes phénomènes. Ces dépôts minéraux sont susceptibles d'obstruer les canaux d'écoulement dans la formation, de polluer les canalisations et les équipements de surface et de bloquer les systèmes de pompage.

Plus généralement, des dépôts minéraux ou des phénomènes de corrosion peuvent apparaître dans différentes conditions d'exploitation d'installations industrielles en contact avec un environnement aqueux.

Pour empêcher ou ralentir ces phénomènes, des additifs sont injectés, par exemple dans la formation souterraine ou dans les puits de gaz ou de pétrole. Un exemple de tels additifs est constitué des inhibiteurs de dépôts minéraux et des inhibiteurs de corrosion. L'injection d'un fluide contenant un inhibiteur capable de prévenir la formation des cristaux problématiques, d'empêcher leur croissance ou de les disperser permet ainsi de contrer les inconvénients précités et d'éviter ou de retarder le démontage des installations en vue de leur nettoyage.

Le dosage (détection et/ou quantification) de ces inhibiteurs constitue toutefois un aspect essentiel de la production d'hydrocarbures, afin de s'assurer qu'ils sont présents en quantité suffisante pour remplir leur fonction et, en cas de besoin, d'injecter en temps utile une quantité supplémentaire d'inhibiteur, ajustée pour tenir compte des contraintes économiques du procédé et de son impact environnemental.

Les méthodes utilisées actuellement pour doser ces inhibiteurs sont souvent peu précises et/ou longues et nécessitent des appareillages souvent insuffisamment adaptés aux conditions d'exploitation. Un des exemples de ces méthodes est le dosage d'inhibiteurs de corrosion par le méthyl orange. Bien que cette technique présente une grande souplesse, elle manque cruellement de fiabilité et présente une incertitude relative très importante sur les résultats.

Pour le dosage de molécules inhibitrices de dépôts minéraux, une analyse chimique précise est nécessaire sur un certain nombre d'éléments spécifiques d'une des molécules utilisées (mesure du taux d'azote ou de phosphore par exemple, méthode dite Hyamine pour les polymères). Ces mesures peuvent être réalisées soit par spectrométrie de masse et/ou par séparation et concentration à l'aide de dispositifs analytiques tels que la chromatographie en phase liquide à haute performance (HPLC). Ces techniques sont à la fois complexes et difficiles à mettre en place.

La mise au point d'une méthode de détection fiable et rapide se heurte en outre au fait que ces additifs ou inhibiteurs sont généralement si efficaces qu'ils ne sont présents dans le fluide injecté qu'à hauteur de quelques ppm, et par la présence dans le fluide constitué des eaux d'exploitation d'une diversité de composés tels que des sels et des résidus organiques. Du fait de la présence de ces composés, le fluide complexe produit présente notamment une fluorescence intrinsèque qui empêche la détection des additifs ou inhibiteurs, éventuellement marqués par une sonde fluorescente, à l'aide des techniques traditionnelles de fluorescence. Enfin, les sites de production sont généralement situés dans des lieux reculés, éloignés des laboratoires d'analyse locaux, ce qui constitue une contrainte supplémentaire.

Il serait donc souhaitable de pouvoir doser ces additifs ou inhibiteurs directement sur site, dans les eaux de production, à l'aide d'une méthode simple, fiable et précise, utilisable sur une diversité d'inhibiteurs de dépôts minéraux et de corrosion et pouvant être mise en œuvre à l'aide d'appareils peu encombrants pour pouvoir être déplacés aisément.

Les inventeurs ont démontré que ces besoins pouvaient être satisfaits en mélangeant les fluides à analyser, susceptibles de contenir ces additifs, à une solution révélatrice comprenant au moins un ion lanthanide et au moins un agent chélateur de l'ion lanthanide et en utilisant la méthode de fluorescence en temps résolu. Cette méthode permet en effet de s'affranchir de la fluorescence naturelle des eaux d'exploitation, qui présente des temps très courts d'émission, et de ne récolter que la lumière émise après un délai de quelques microsecondes à une milliseconde, de préférence de 100 microsecondes à une milliseconde, résultant de la fluorescence des inhibiteurs ainsi marqués. En outre, la pré-complexation des ions lanthanides avec un agent chélateur permet d'améliorer considérablement la sensibilité de détection des additifs ou inhibiteurs, et ce, même malgré l'augmentation de la fluorescence des ions lanthanides liée à la présence de l'agent chélateur. La présente méthode permet également de détecter des additifs ou inhibiteurs à faible pouvoir complexant (notamment certains inhibiteurs aminés de corrosion) ou excitable facilement dans le proche UV/visible (comme l'Europium).

Cette méthode a en outre pour avantage d'identifier spécifiquement le type d'additif, par exemple d'inhibiteur (de dépôts minéraux ou de corrosion), sans étape de marquage préalable et même lorsqu'il est présent dans un fluide complexe formé par les eaux de production dans le milieu pétrole, en fonction de sa signature optique, en exploitant simultanément les spectres d'excitation et d'émission et les durées de vie des signaux émis.

### RESUME DE L'INVENTION

La présente invention a pour objet une méthode de détection d'additifs dans un fluide aqueux comprenant:
a. le mélange d'une solution révélatrice comprenant un cation lanthanide et un agent chélateur des lanthanides, avec un échantillon du fluide aqueux à analyser comprenant éventuellement au moins un additif, dans des conditions permettant la complexation du lanthanide par l'agent chélateur et par l'additif éventuellement présent, en quoi l'agent chélateur est choisi parmi la diaminopyridine, l'imidazoline, le polyanhydride maléique hydolysé, l'acide oxalique, l'acetylacetonate, le thiodiacétate, l'acide nitrilotriacétique (NTA), ou leurs dérivés;
b. La détection et, le cas échéant, la quantification, de la variation de fluorescence liée à la présence éventuelle de l'additif dans le fluide aqueux par fluorescence en temps résolu.

Elle a également pour objet une solution révélatrice pour la détection d'additifs et notamment d'inhibiteurs de dépôts minéraux ou de corrosion, comprenant :
(i) un cation lanthanide, par exemple Eu3+,
(ii) un agent chélateur des lanthanides, par exemple choisi parmi la diaminopyridine, l'imidazoline, le polyanhydride maléique hydolysé, les poly-carboxiliques, l'acide oxalique, l'acetylacetonate, le thiodiacetate, ou leurs dérivés, l'EDTA, l'acide nitrilotriacétique (NTA) ;
(iii) des ions chlorure à plus de 1 g/L ;
(iv) le cas échéant, un composé chimique utilisé dans la fabrication de solution tampon à plus de 1g/L, par exemple l'HEPES ;
le rapport de concentration entre l'agent chélateur et le lanthanide étant compris entre 1 :10 et 10 :1, de préférence entre 1 :3 et 3 :1.

### DESCRIPTION DETAILLEE DE MODES DE REALISATIONS

La méthode selon l'invention comprend essentiellement :
- le mélange d'une solution révélatrice comprenant un cation lanthanide et un agent chélateur des lanthanides, avec un échantillon du fluide aqueux à analyser comprenant éventuellement au moins un additif, dans des conditions permettant la complexation du lanthanide par l'agent chélateur et par l'additif, et
- la détection et, le cas échéant, la quantification de la variation de fluorescence liée à la présence éventuelle de l'additif dans le fluide aqueux par fluorescence en temps résolu. La méthode selon l'invention est définit dans la revendication 1.

### Solution révélatrice pour la mise en œuvre de la méthode de détection d'additifs

La mise en œuvre de la méthode de détection comprend l'utilisation d'une solution révélatrice comprenant au moins un cation lanthanide et au moins un agent chélateur des lanthanides.

Par « agent chélateur des lanthanides », on entend un ion ou une molécule portant des fonctions chimiques lui permettant de se lier à un ou plusieurs atomes ou ions centraux dudit lanthanide et dont l'interaction lanthanide/ligand ainsi formée est supérieure à l'interaction lanthanide/eau et de préférence supérieure à l'interaction lanthanide/Chlorure, permettant ainsi une pré-complexation des ions lanthanides en diminuant le nombre de molécules d'eau présentes dans la sphère de coordination du lanthanide, il en résulte une diminution de l'effet quenching de l'eau sur la fluorescence de l'ion lanthanide (DeW. Horrocks et al., JACS 1979 101 :2, 334-340).

Dans la methode de la presente invention l'agent chélateur est choisi parmi la diaminopyridine, l'imidazoline, le polyanhydride maléique hydrolysé, les polyacides carboxyliques, l'acide oxalique, l'acétylacétonate, le thiodiacétate, l'acide nitrilotriacétique (NTA), leurs dérivés ou leurs mélanges.

Dans un mode de réalisation spécifique, la solution révélatrice comprend en outre au moins 1g/L d'ions chlorures, et de préférence une concentration en ions chlorures comprise entre 10 et 50g/L.

Dans un autre mode de réalisation particulier pouvant être combiné avec les précédents, la solution révélatrice comprend en outre au moins 1g/L d'un composé à fort pouvoir tampon, utilisé dans la fabrication de solution tampon, et permettant de maintenir la valeur du pH après ajout de 1/10 d'eau à analyser dans un intervalle de pH inférieur à 0,5 unité, par exemple l'acide 4-(2-hydroxyéthyl)-1-pipérazine éthane sulfonique (HEPES).

Le rapport de concentration dans la solution révélatrice entre l'agent chélateur et le lanthanide est compris entre 1 :10 et 10 :1, par exemple entre 1 :3 et 3 :1.

Le cation lanthanide utilisable dans la solution révélatrice peut être choisi parmi les éléments de numéro atomique 57 (lanthane) à 71 (lutétium), tels que Pr, Nd, Sm, Eu, Tb, Dy, Ho, Er, Tm et Yb, ainsi que leurs mélanges. La présente méthode est particulièrement adaptée pour une solution révélatrice comprenant l'Europium, dont les variations de fluorescence sont plus difficilement détectables en l'absence de l'agent chélateur présent dans la solution révélatrice. Avantageusement, la mesure de la fluorescence émise par l'Europium peut être effectuée au travers de cellules plastiques jetables, sans la nécessité d'utiliser des cellules transparentes dans l'UV (comme les cellules en quartz ou silice).

L'invention a trait en particulier à une solution révélatrice pour la détection d'additifs et notamment d'inhibiteurs de dépôts minéraux ou de corrosion, comprenant :
i. un cation lanthanide, par exemple Eu3+,
ii. un agent chélateur des lanthanides, par exemple choisi parmi la diaminopyridine, l'imidazoline, le polyanhydride maléique hydolysé, les polyacides carboxyliques, l'acide oxalique, l'acétylacétonate, le thiodiacétate, ou leurs dérivés, l'acide nitrilotriacétique (NTA) ;
iii. des ions chlorure à plus de 1 g/L ;
iv. le cas échéant, un composé chimique utilisé classiquement dans la fabrication des solutions tampon par exemple l'acide 4-(2-hydroxyéthyl)-1-pipérazine éthane sulfonique (HEPES) à plus de 1g/L, par exemple l'HEPES : acide 4-(2-hydroxyéthyl)-1-pipérazine éthane sulfonique;
le rapport de concentration entre l'agent chélateur et le lanthanide étant compris entre 1 :10 et 10 :1, de préférence entre 1 :3 et 3 :1.

Dans un mode de réalisation particulier, la solution révélatrice comprend entre 10 et 1000ppm de EuCl₃.6H₂0, 10 et 5000ppm d'imidazoline et entre 5 et 50g/L de NaCl, à pH compris entre 4 et 8.

Dans un autre mode de réalisation, la solution révélatrice comprend entre 1 et 100 ppm de EuCl₃.6H₂0, 2 et 200ppm de polyanhydride maléique hydrolysé et entre 5 et 50g/L de NaCl, à pH compris entre 4 et 8.

La présente invention vise les solutions révélatrices telles que définies ci-dessus en tant que telles, leurs procédés de préparation, et leurs utilisations dans une méthode de détection d'additifs comme définie ci-dessous.

### Fluide aqueux à analyser, extraction et mélange avec la solution révélatrice

La méthode de détection d'additif comprend l'extraction d'un échantillon (fluide aqueux) à analyser et son mélange avec la solution révélatrice définie ci-dessus.

Les additifs susceptibles d'être présents dans l'échantillon à analyser, et détectables par la méthode de détection selon l'invention, sont notamment ceux présentant au moins une fonction amine.

Dans un mode de réalisation, l'additif ne présente pas de fluorescence intrinsèque. Avantageusement, avec la méthode selon la présente invention, l'additif à détecter n'a pas été couplé à un agent chélateur avant son injection ou un agent luminescent ou autre agent de marquage.

La méthode peut donc être appliquée par exemple à l'analyse et la détection d'additifs chimiques utilisés dans l'industrie chimique ou présents dans l'environnement.

De manière préférée, la méthode vise la détection d'inhibiteur de dépôts minéraux ou de corrosion, en particulier dans un extrait d'un fluide pétrolier contenant de l'eau, par exemple, un extrait d'un puits de production de pétrole ou de gaz. Dans un mode préféré de la méthode selon l'invention, on extrait un échantillon de fluide aqueux à analyser d'un puits de production de pétrole ou de gaz ou d'une eau industrielle prélevée au cours du processus d'exploitation et/ou de fabrication.

Par "inhibiteur de dépôts minéraux", on entend un composé capable d'empêcher ou de ralentir la formation (c'est-à-dire la nucléation et/ou la croissance) de cristaux de sels minéraux choisis notamment parmi : le carbonate de calcium, le sulfate de calcium, le sulfate de baryum, le sulfate de strontium, les sulfures de zinc, de plomb et de fer et leurs mélanges. Les inhibiteurs de dépôts minéraux détectables selon la méthode de l'invention sont notamment:
- les polyphosphates, tels que le tripolyphosphate de sodium (STPP), l'hexamétaphosphate de sodium (SHMP),
- les organophosphonates, tels que l'acide amino tri(méthylène phosphonique) ou AMP, l'acide 1-hydroxyéthylidène-1,1-diphosphonique ou HEDP, l'acide éthylènediamine tétra(méthylène phosphonique) ou EDTMP, l'acide hexaméthylènediamine tétra(méthylène phosphonique) ou HMTMP, l'acide diéthylènetriamine penta(méthylène phosphonique) ou DETMP, l'acide hydroxyéthylamino-di(méthylènephosphonique) (HEMPA),
- les polyacides carboxyliques tels que l'acide 2-phosphonobutane 1,2,4-tricarboxylique ou PBTC, le poly(acide acrylique) ou PAA, le poly(acide méthacrylique) ou PMAA, le poly(acide maléique) ou PMA,
- les polymères à fonction acide sulfonique, tels que les copolymères d'acide styrène sulfonique et d'acide (poly)carboxylique, en particulier les copolymères d'acide styrène sulfonique et d'acide maléique, les copolymères d'acide styrène sulfonique et de (poly)amidoamine, les homo- et copolymères de vinylsulfonate, en particulier les copolymères de vinylsulfonate, de styrène et d'anhydride maléique, les alkyldiphényléther sulfonates et les copolymères d'acide acrylamidométhylpropane sulfonique (AMPS), d'acide maléique et d'acide acrylique,
- l'acide polyphosphinocarboxylique (PPCA) éventuellement sulfonaté,
- la polyéthylèneimine (PEI),
- les polymères siliconés, en particulier les polydiméthylsiloxanes, fonctionnalisés par des groupements amines, et
- les copolymères à base d'ammonium quaternaire, tels que les copolymères d'acrylamide, d'ammonium quaternaire et éventuellement d'acrylate et les copolymères d'acrylamide, de sel de diallyldiméthylammonium et éventuellement d'acrylate.

Par "inhibiteur de corrosion", on entend un composé capable de réduire la vitesse de corrosion d'un matériau, typiquement un métal ou un alliage métallique, due à l'action sur ce matériau d'un oxydant tel que le dioxygène ou l'ion H⁺. Des exemples d'inhibiteurs de corrosion sont notamment les mono-, di- et triamines telles que la cyclohexylamine, l'hexylamine, la morpholine, l'imidazoline ou l'octadécylamine, ainsi que les amino-alcools tels que le diéthylaminoéthanol, les ammoniums quaternaires et les bétaïnes.

Dans la méthode selon l'invention, le volume extrait de l'échantillon à analyser (par exemple extrait de puits de pétrole ou de gaz) peut être par exemple compris entre 0,1 mL et 1 litre, 5 litres, 10 litres ou plus.

Le volume extrait peut être traité avant analyse, par exemple par acide/base/oxydation/précipitation ou par des étapes de filtration ou sédimentation afin d'éliminer certains composés indésirables, avant le mélange avec la solution révélatrice.

On mélange cet échantillon (par exemple extrait de puits de pétrole ou de gaz) avec la solution révélatrice, par exemple dans des proportions de 1 :100 à 100 :1 vol/vol entre le volume de l'échantillon à analyser et le volume de solution révélatrice et de préférence compris entre 1 :20 et 1 :5, par exemple autour de 1 :10.

Les ions lanthanides pré-complexés avec les agents chélateurs présents dans la solution révélatrice forment des complexes avec les additifs éventuellement présents dans l'échantillon (par exemple inhibiteur de dépôt ou de corrosion). Il en résulte une diminution de l'effet quenching des molécules d'eau autour des cations lanthanides et donc une augmentation de la fluorescence des cations lanthanides.

### Détection des additifs dans l'échantillon à analyser

Pour la détection des additifs dans l'échantillon à analyser avec la méthode selon l'invention, on mesure la variation de fluorescence entre ce mélange comprenant des ions lanthanides éventuellement complexés avec un additif et un mélange contrôle (par exemple ne contenant pas d'additif ou contenant une quantité connue d'additif), par fluorescence en temps résolu.

De fait de la diminution de l'effet quenching de l'eau sur le lanthanide en présence de l'additif, la variation de fluorescence par rapport à une solution de référence sans additif (ou une quantité connue d'additif) est ainsi directement et spécifiquement reliée à la présence d'additif dans le fluide à analyser.

La comparaison des caractéristiques d'émission, d'excitation et/ou de durée de vie des ions lanthanides libres et des ions lanthanides complexés permet ainsi de détecter et, le cas échéant de quantifier les inhibiteurs présents dans le fluide extrait.

Selon l'invention, l'additif (par exemple l'inhibiteur de dépôt ou de corrosion) est détecté, et son taux quantifié, en utilisant une méthode de fluorescence en temps résolu qui est notamment décrite dans l'article "Ultrasensitive bioanalytical assays using time resolved fluorescence detection", Pharmacol. Ther. Vol. 66(2), pp. 207-35, 1995. Celle-ci repose sur l'application d'un délai, dit délai d'intégration, entre l'excitation de l'échantillon à analyser et la mesure du signal émis, de manière à s'affranchir des fluorescences parasites à durée de vie courte. Cette méthode peut être mise en œuvre à température ambiante, notamment à l'aide d'un appareil de type Cary Eclipse de la société Agilent.

La longueur d'onde d'excitation peut être comprise entre 200 et 600 nm et la longueur d'onde d'émission peut être comprise entre 300 et 800 nm. Le délai d'intégration peut être compris entre 0,001 et 10 ms, de préférence entre 0,01 et 5 ms, plus préférentiellement entre 0,1 et 3 ms. Dans certains cas, plus ce délai est long, meilleur est le rapport signal / bruit, ce qui améliore la fiabilité de la mesure. La durée de récolte des photons peut aller de 5 à 10 ms, par exemple.

Cette méthode peut être appliquée de différentes manières. Il est ainsi possible de comparer l'intensité d'émission de l'échantillon testé avec celles obtenues à différentes concentrations d'inhibiteur, pour en déduire la concentration de l'inhibiteur dans l'échantillon. En variante, il est possible de détecter plusieurs inhibiteurs dans l'échantillon testé en mesurant la vitesse de décroissance du signal émis par l'échantillon, ou demi-vie, et en comparant les valeurs obtenues avec celles connues pour les différents additifs à détecter.

### FIGURES

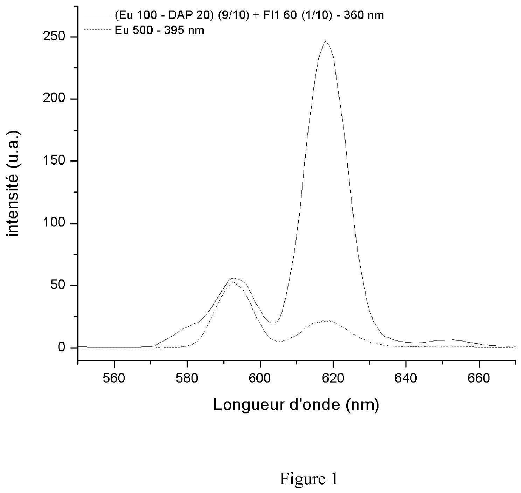

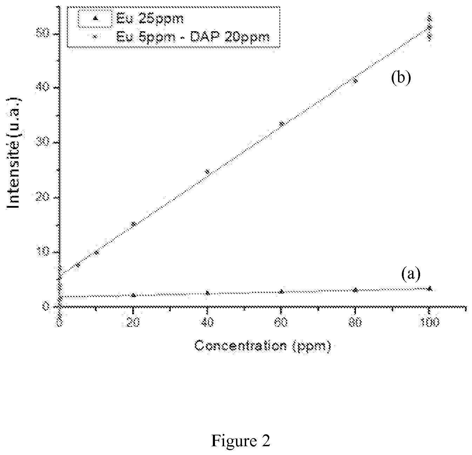

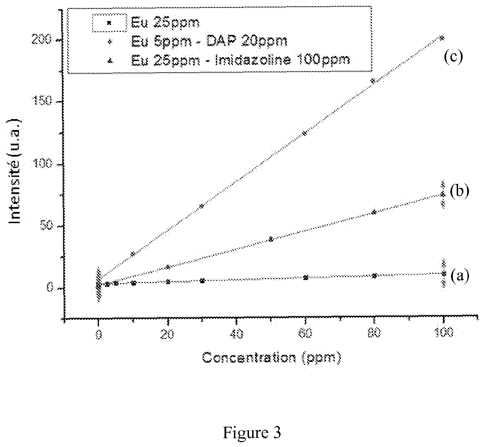

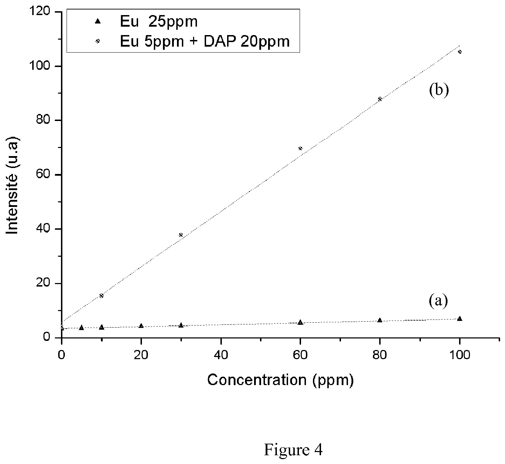

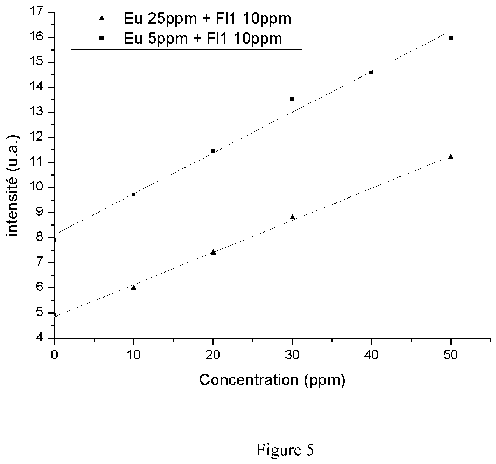

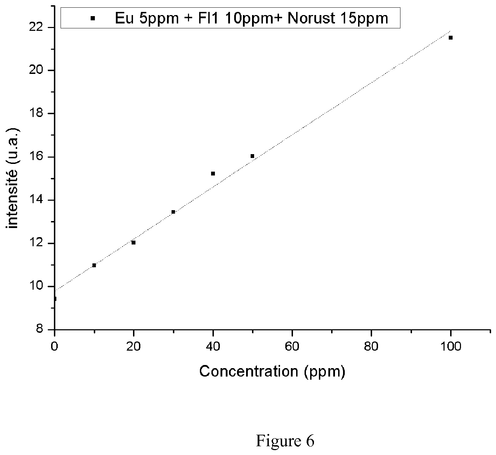

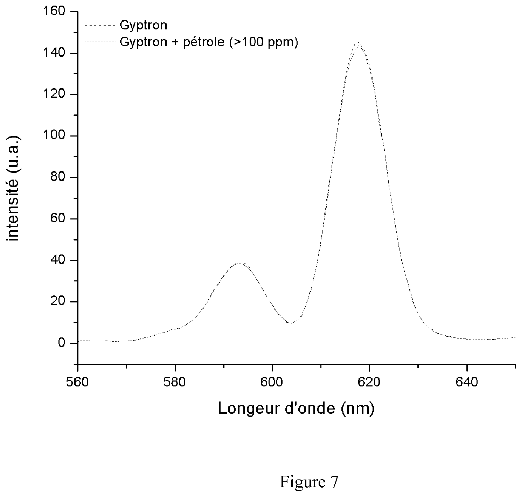

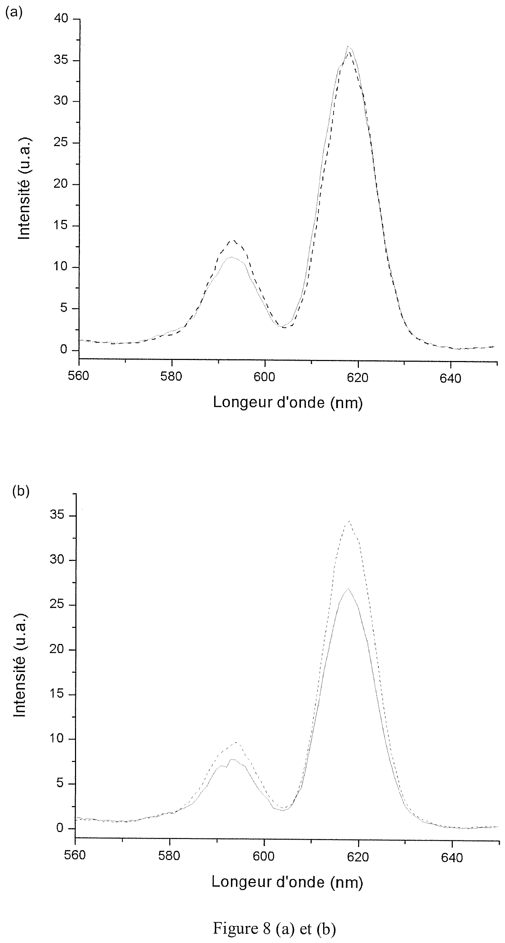

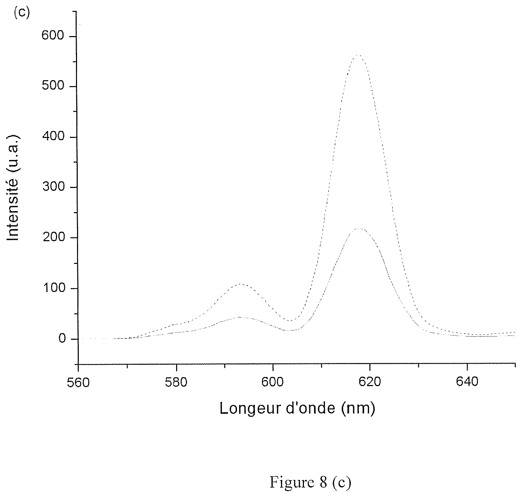

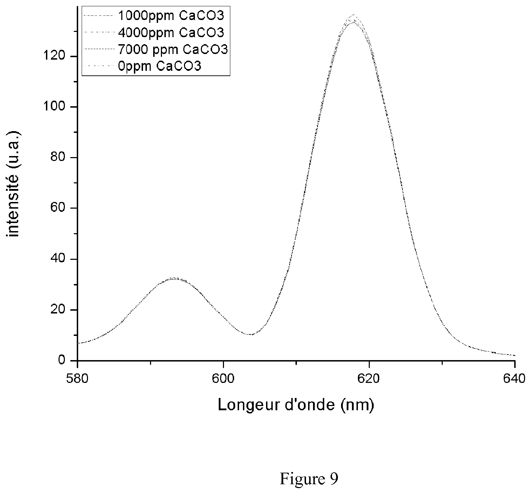

**La** **Figure 1** présente un spectre d'émission de l'europium libre (courbe du bas, Eu 500-395nm) et de l'europium complexé au FL1/DAP (Courbe du haut) (Eu 100 - DAP 20) (9/10) et FL1 60 (1/10) - 360nm
**La** **Figure 2** présente une courbe de calibration du Gyptron SA3470 par l'europium avec (a) la solution Eu-Light (λ_{exc} = 395nm) et (b) Eu-LD520 (λ_{exc} = 360 nm)
**La** **Figure 3** présente les courbes de calibration du Fl1 par l'Europium (a) Eu-light (λ_{exc} = 395nm), (b) Eu-IM25100 (λ_{exc} = 360 nm) et (c) Eu-LD520 (λ_{exc}=360nm).
**La** **Figure 4** présente les courbes de calibration du ZFD avec (a) Eu-light (λ_{exc} = 395nm) et (b) Eu-LD520 (λ_{exc} = 360 nm)
**La** **Figure 5** présente les courbes de calibration du Nalco EC1621A (λ_{exc} = 350nm) avec (a) Eu-FL510 et (b) Eu-F12510
**La** **Figure 6** présente les courbes de calibration du Norust9711 (λ_{exc} = 350nm) avec Eu-FL510
**La** **Figure 7** illustre les spectres d'émission (λ_{exc} = 360nm) du complexe Gyptron-Eu avec et sans pétrole.
**La** **Figure 8** présente les spectres d'émission du complexe Gyptron-Eu, la quantification par la solution Eu-LD20 à (a) 100ppm de sel d'europium (Eu-LD10020), (b) 50ppm de sel d'europium (Eu-LD5020) et (c) 5ppm de sel d'europium (Eu-LD520) pour une eau à faible salinité (courbe en pointillé, échantillon 1) et une eau à forte salinité (courbe en continu, échantillon 2).
**La** **Figure 9** présente les spectres d'émission du complexe Fl1-Eu Quantifié par la solution Eu-LD10020 en présence d'une quantité variable de CaCO₃.

La présente invention sera mieux comprise à la lumière des exemples non limitatifs suivants, qui sont donnés à titre purement illustratif et n'ont pas pour but de limiter la portée de cette invention qui est définie par les revendications annexées.

### EXEMPLES

### I- Préparation de solutions d'additifs et des solutions révélatrices

### Préparation des eaux salines :

### Eau synthétique (Echantillon 1)

**Tableau 1 : composition saline de l'eau synthétique échantillon 1**

| | Echantillon 1 (g/L) |
|---|---|
| NaCl | 96.00 |
| CaCl₂ | 134.65 |
| MgCl₂.6H₂O | 26.40 |
| KCl | 5.70 |
| BaCl₂.2H₂O | 0.55 |
| SrCl₂.6H₂O | 1.90 |

**Tableau 2 : Composition de l'eau synthétique (échantillon 2)**

| | Echantillon 2 (g/L) |
|---|---|
| NaCl | 90.44 |
| CaCl₂ | 18.66 |
| MgCl₂.6H₂O | 1.08 |
| KCl | 10.59 |
| BaCl₂.2H₂O | 2.47 |
| SrCl₂.6H₂O | 2.24 |

### Préparation d'une solution mère d'europium :

250 mg de trichlorure d'europium hexahydraté (CAS : 13759-92-7 ; EuCl3.6H2O) sont pesés dans un flacon de 500 mL. Par la suite, 500 mL d'eau ultra-pure sont ajoutés. L'échantillon est placé sous agitation environ 1 h. Une solution à 500 ppm de EuCl3.6H2O est obtenue.

### Préparation d'une solution mère d'HEPES (tampon):

2,98 g d'HEPES (CAS : 7365-45-9; C8H18N2O4S ; Sigma Aldrich) sont pesés dans un flacon de 250 mL. Par la suite, 250 mL d'eau ultra-pure sont ajoutés. L'échantillon est placé sous agitation environ 1 h. Une solution à 11.92 g/L d'HEPES est obtenue.

### Préparation d'une solution mère d'agent chélateur diaminés amplificateurs (DAP)

1 g de 2,5-diaminopyridine dihydrochloride (DAP) (CAS : 26878-35-3 ; C5H7N3.2HCl ; Alfa Aesar) sont pesés dans un flacon de 100 mL. Par la suite, 100 mL d'eau ultra-pure sont ajoutés. L'échantillon est placé sous agitation environ 1 h. Une solution rouge à 10 g/L de DAP est obtenue.

### Préparation d'une solution mère d'agents chélateurs carboxylés amplificateurs (Fl10)

200 mg de sel de poly (acide 4-styrènesulfonique, acide co-maléique) de sodium (CAS : 68037-40-1 ; [CH2CH(C6H4SO3R)]x[CH(CO2R)CH(CO2R)]y R = H/Na ; Sigma Aldrich) sont pesés dans un flacon de 100 mL. Par la suite, 100 mL d'eau ultra-pure sont ajoutés. L'échantillon est placé sous agitation environ 1 h. Une solution à 2 g/L de complexant est obtenue.

### Préparation d'une solution de référence d'inhibiteur de corrosion Nalco EC1621A

1 g de solution Nalco EC1621A (fabriqué par Nalco, Ecolab Europe Gmbh Suisse) est pesé dans un flacon de 100 mL. Par la suite, 100 mL d'eau ultra-pure sont ajoutés. L'échantillon est placé sous agitation environ 1 h. Une solution à 10 g/L de Nalco EC1621A est obtenue.

### Préparation d'une solution de référence d'inhibiteur de corrosion Norust 9711

1 g de solution Norust 9711 (fabriqué par CECA, Groupe Arkema France) est pesé dans un flacon de 100 mL. Par la suite, 100 mL d'eau ultra-pure sont ajoutés.

L'échantillon est placé sous agitation environ 1 h. Une solution à 10 g/L de Norust 9711 est obtenue.

### Préparation de la solution révélatrice Eu-light sans agent chélateur

25 mL de la solution mère d'Hepes sont placés dans un flacon de 500 mL. Par la suite, 50 mL de la solution mère sont ajoutés avec 425 mL d'eau ultra-pure et 10 g de chlorure de sodium. Le pH est alors ajusté à 6,5 avec une solution de NaOH 1M. Une solution révélatrice à pH 6.5 avec 25 ppm de EuCl3.6H2O et 20 g/L de NaCl est obtenue.

### Préparation d'une solution d'inhibiteur de corrosion Gyptron SA3470 dans une eau saline (solution TEST1)

1 g de la solution commerciale de Gyptron SA3470 (fabriqué par Champion Technologies Ltd, UK) est pesé dans un flacon de 100 mL. Par la suite, 100 mL d'eau saline (eau synthétique, échantillon 1) sont ajoutés. L'échantillon est placé sous agitation environ 1 h. 5 mL de la solution préparée sont prélevés puis placés dans un flacon de 500 mL. Enfin, 495 mL d'eau saline (eau synthétique, échantillon 1) sont ajoutés. Une solution à 100 ppm de solution commerciale de Gyptron SA3470 dans une eau saline synthétique (échantillon 1).

### Préparation d'une solution d'inhibiteur de dépôt Fl1 dans une eau saline (solution TEST2)

1 g d'inhibiteur de dépôt Fl1 est pesé dans un flacon de 100 mL. Par la suite, 100 mL d'eau saline (eau synthétique, échantillon 2) sont ajoutés. L'échantillon est placé sous agitation environ 1 h. 5 mL de la solution préparée sont prélevés puis placés dans un flacon de 500 mL. Enfin, 495 mL d'eau saline échantillon 2 synthétique sont ajoutés. Une solution à 100 ppm de Fl1 dans une eau saline est obtenue.

### Préparation d'une solution d'inhibiteur de dépôt ZFD dans une eau saline (solution TEST3)

1 g de ZFD est pesé dans un flacon de 100 mL. Par la suite, 100 mL d'eau saline (eau synthétique, échantillon 2) sont ajoutés. L'échantillon est placé sous agitation environ 1 h. 5 mL de la solution préparée sont prélevés puis placés dans un flacon de 500 mL. Enfin, 495 mL d'eau saline synthétique (échantillon 2) sont ajoutés. Une solution à 100 ppm de ZFD dans une eau saline est obtenue.

### Préparation d'une solution d'inhibiteur de corrosion Nalco EC1621A dans une eau saline (solution TEST4)

1 mL de la solution de référence au Nalco EC1621A sont placés dans un flacon de 100 mL. Par la suite, 99 mL d'eau saline dsynthétique (échantillon 2) sont ajoutés. Une solution à 100 ppm de Nalco EC1621A dans une eau saline est obtenue.

### Préparation d'une solution d'inhibiteur de corrosion Norust 9711 dans une eau saline

1 mL de la solution de référence au Norust 9711 sont placés dans un flacon de 100 mL. 99 mL d'eau saline (eau synthétique, échantillon 2) sont ajoutés. Une solution à 100 ppm de Norust 9711 dans une eau saline est obtenue.

### Exemple 1

### Préparation de la solution révélatrice amplifiée au pré-complexant imidazoline : Eu-IM25100

Pour une solution à 25 ppm de sel d'europium (Eu-IM25100) : 25 mL de la solution mère d'Europium sont placés dans un flacon de 500 mL. Par la suite, 50 mL de la solution mère d'HEPES ainsi que 420 mL d'eau ultra-pure sont ajoutés. 5 mL d'une solution à 10 g/L d'une solution commerciale d'imidazoline et 10 g de chlorure de sodium sont ensuite ajoutés. Le pH est alors ajusté à 6,5 avec une solution de NaOH 1M. Une solution révélatrice à pH 6.5 avec 25 ppm de EuCl3.6H2O, 100 ppm d'une solution commerciale d'imidazoline et 20 g/L de NaCl est obtenue.

### Exemple 2

### Préparation de la solution révélatrice amplifiée au pré-complexant diaminé (DAP): Eu-LD20

La quantité de sel d'europium est ajustée selon la concentration souhaitée. Pour une solution à 5 ppm de sel d'europium (Eu-LD520) : 5 mL de la solution mère d'Europium sont placés dans un flacon de 500 mL. Par la suite, 50 mL de la solution mère d'HEPES ainsi que 444 mL d'eau ultra-pure sont ajoutés. 1 mL de la solution mère d'agent chélateur DAP et 10 g de chlorure de sodium sont ensuite ajoutés. Le pH est alors ajusté à 6,5 avec une solution de NaOH 1M. Une solution révélatrice à pH 6.5 avec 5 ppm de EuCl3.6H2O, 20 ppm de DAP et 20 g/L de NaCl est obtenue.

### Exemple 3

### Préparation de la solution révélatrice amplifiée au pré-complexant carboxylé : Eu-Fl10

La quantité de sel d'europium est ajustée selon la concentration souhaitée. Pour une solution à 5 ppm de sel d'europium (Eu-FL510) : 5 mL de la solution mère d'Europium sont placés dans un flacon de 500 mL. Par la suite, 50 mL de la solution mère d'HEPES ainsi que 442,5 mL d'eau ultra-pure sont ajoutés. Enfin, 2,5 mL de la solution mère de Fl10 et 10 g de chlorure de sodium sont ajoutés. Le pH est alors ajusté à 6,5 avec une solution de NaOH 1M.

Une solution révélatrice à pH 6.5 avec 5 ppm de EuCl3.6H2O, 10ppm de pré-complexant carboxylé et 20 g/L de NaCl est obtenue.

### II-Méthodes de quantification des additifs

### Quantification des additifs par fluorescence en temps résolution (FTR) :

Les mesures sont effectuées sur un spectrofluorimètre Agilent Cary Eclipse. Elles sont effectuées dans des cuvettes en PMMA (référence) de 4 mL. Les concentrations en solutions révélatrices sont fixées pour des concentrations décroissantes d'additif formulé une eau synthétique échantillon 1. Le temps de vie de luminescence des terres rares augmente avec la diminution du nombre de molécules d'eau dans leur sphère de coordination. La complexion des terres rares par des additifs tels que les inhibiteurs permet ainsi leur détection et leur quantification. Cette complexation est caractérisée par une augmentation de l'intensité d'émission et une inversion du rapport des pics I595nm/I615nm (Figure 1).

Les temps de vie de fluorescence de ces complexes sont typiquement de l'ordre de la milliseconde. Cette propriété permet notamment de les distinguer de la fluorescence des composés organiques qui est de l'ordre de la microseconde.

Les complexes d'europium possèdent quatre pics notables dans le visible : 536, 595, 614 et 650 nm. Les limites de l'appareillage (perte de sensibilité pour λem > 600 nm) nous ont conduites à quantifier ces entités via l'émission à 614 nm. L'intensité du pic est relative à la concentration, au degré de complexation et aux conditions de détection.

### Dosage par TRF avec la solution révélatrice d'europium Eu-light

Les longueurs d'onde d'excitation et d'émission sont respectivement réglées à 395 nm et 617 nm. Le « delay time » est réglée à 0,2 ms et le « gate time » à 1 ms.

### Dosage par TRF avec la solution révélatrice Eu-IM25100 (exemple 1) et Eu-LD20 (exemple 2)

Les longueurs d'onde d'excitation et d'émission sont respectivement réglées à 360 nm et 617 nm. Le delay time est réglée à 0,2 ms et le gate time à 1 ms.

### Dosage par TRF avec la solution révélatrice Eu-Fl10 (exemple 3)

Les longueurs d'onde d'excitation et d'émission sont respectivement réglées à 350 nm et 617 nm. Le delay time est réglée à 0,1 ms et le gate time à 1 ms.

### II-1 Détection des inhibiteurs de dépôt

### TEST 1 : Procédure de quantification du Gyptron SA3470

Plusieurs échantillons sont préparés à partir de la solution TEST1 afin d'obtenir une gamme de concentration entre 0 et 100 ppm dans l'échantillon 1. Ces échantillons sont dosés par TRF selon 2 méthodes :
1 mL de chaque échantillon sont placées dans des flacons de 10 mL en présence de 9 mL de la solution Eu-light (Europium seul). Après une heure, 2 mL de ces nouvelles solutions sont analysés selon les conditions expérimentales définies ci-dessus.

1 mL de chaque échantillon sont placées dans des flacons de 10 mL en présence de 9 mL de la solution de l'exemple 2.

Après une heure, 2 mL de ces nouvelles solutions sont analysés selon les conditions expérimentales définies ci-dessus.

Les courbes de calibrations obtenues sont présentées en Figure 2. Lorsque la méthode est employée, les droites montrent un effet d'exaltation de la fluorescence accompagné d'une augmentation de la sensibilité sur la mesure avec conservation de la linéarité.

### TEST2 : Procédure de quantification du Fl1

Plusieurs échantillons sont préparés à partir de la solution TEST2 afin d'obtenir une gamme de concentration entre 0 et 100 ppm dans l'eau saline synthétique (échantillon 2). Ces échantillons sont dosés par TRF selon 2 méthodes :
1 mL de chaque échantillon sont placés dans des flacons de 10 mL en présence de 9 mL de la solution Eu-Light (Europium seul). Après une heure, 2 mL de ces nouvelles solutions sont analysés selon les conditions définies ci-dessus.

1 mL de chaque échantillon sont placés dans des flacons de 10 mL en présence de 9 mL de la solution de l'exemple 1. Après une heure, 2 mL de ces nouvelles solutions sont analysés selon les conditions expérimentales définies ci-dessus.

0,5 mL de chaque échantillon sont placés dans des flacons de 10 mL en présence de 9,5 mL de la solution de l'exemple 2. Après une heure, 2 mL de ces nouvelles solutions sont analysés selon les conditions expérimentales définies ci-dessus.

Les courbes de calibrations obtenues sont présentées en Figure 3. Lorsque la méthode est employée, les droites montrent un effet d'exaltation de la fluorescence accompagné d'une augmentation de la sensibilité sur la mesure avec conservation de la linéarité pour les deux solutions révélatrices Exemple 1 et Exemple 2.

### Procédure de quantification du ZFD

Plusieurs échantillons sont préparés à partir de la solution TEST3 afin d'obtenir une gamme de concentration entre 0 et 100 ppm dans l'eau saline synthétique de l'échantillon 2. Ces échantillons sont dosés par TRF selon 2 méthodes :
1 mL de chaque échantillon sont placées dans des flacons de 10 mL en présence de 9 mL de la solution Eu-light (Europium seul). Après une heure, 2 mL de ces nouvelles solutions sont analysés selon les conditions expérimentales définies ci-dessus.

1 mL de chaque échantillon sont placées dans des flacons de 10 mL en présence de 9 mL de la solution de l'exemple 2. Après une heure, 2 mL de ces nouvelles solutions sont analysés selon les conditions expérimentales définies ci-dessus.

Les courbes de calibrations obtenues sont présentées en Figure 4. Lorsque la méthode est employée, les droites montrent un effet d'exaltation de la fluorescence accompagné d'une augmentation de la sensibilité sur la mesure avec conservation de la linéarité.

### II-2 Inhibiteurs de corrosion

### Procédure de quantification du Nalco EC1621A

Plusieurs échantillons sont préparés à partir de la solution TEST4 afin d'obtenir une gamme de concentration entre 0 et 50 ppm dans l'eau saline synthétique de l'échantillon 2.

2 mL de chaque échantillon sont placées dans un flacon de 10 mL en présence de 8 mL de la solution de l'exemple 3 (Eu-Fl510 et Eu-Fl2510). Après une heure, 2 mL de ces échantillons sont analysés selon les conditions expérimentales définies ci-dessus.

Les courbes de calibrations obtenues sont présentées en Figure 5.

### Procédure de quantification du Norust 9711

Plusieurs échantillons sont préparés à partir de la solution TEST5 afin d'obtenir une gamme de concentration entre 0 et 100 ppm dans l'eau saline synthétique de l'échantillon 2.

2 mL de chaque échantillon sont placées dans un flacon de 10 mL en présence de 8 mL de la solution de l'exemple 3 (Eu-Fl510). Après une heure, 2 mL de cet échantillon sont analysés selon les conditions expérimentales définies ci-dessus.

Les courbes de calibrations obtenues sont présentées en Figure 6.

### III-Effet des différents paramètres sur la mesure

### Interférences du pétrole sur la mesure

Il s'agit d'évaluer l'influence de l'huile sur les données spectrales lors de la quantification du GyptronSA3470. Tout d'abord une solution à 40 ppm de Gyptron est préparée par dilution de la solution TEST1. Enfin, environ 100 ppm de pétrole sont introduites dans cette nouvelle solution.

1 mL de cet échantillon est placé dans un flacon de 10 mL en présence de 9 mL de la solution de l'exemple 2 à 100 ppm de sel d'europium (Eu-LD10020). Après une heure, 2 mL de cette nouvelle solution sont analysés selon les conditions expérimentales définies ci-dessus. Les spectres obtenus sont présentés en Figure 7.

Les spectres d'émissions montrent moins de 1% de variation entre les intensités des signaux du pic à 617 nm de l'europium.

### Influence de la salinité

Il s'agit d'évaluer l'influence de la variation de salinité sur les données spectrales lors de la quantification du GyptronSA3470. Deux solutions à 60 ppm de Gyptron sont préparées par dilution de la solution TEST1 dans une eau de mer synthétique (échantillon 1) présentant une forte salinité et une eau de mer synthétique de plus faible salinité (échantillon 2).

1 mL de chaque échantillon est placé dans des flacons de 10 mL en présence de 9 mL de la solution de l'exemple 1 à :
5 ppm de sel d'europium (Eu-LD520)
50 ppm de sel d'europium (Eu-LD5020)
100 ppm de sel d'europium (Eu-LD10020)

Après une heure, 2 mL de ces nouvelles solutions sont analysés selon les conditions expérimentales définies ci-dessus. Les spectres obtenus sont présentés en Figure 8. La quantité de sel d'europium de la solution révélatrice Eu-LD20 a été ajustée afin de minimiser l'effet de sel. En effet, en présence d'une plus grande concentration d'ions europium (de 5 à 100 ppm de sel) les signaux d'émissions sont similaires quel que soit la salinité du milieu. En revanche, pour une concentration de 5 ppm en sel d'europium, la différence entre les deux signaux d'émission (pic à 617 nm) se chiffre à 60 % d'écart.

### Influence des ions carbonates

Il s'agit d'évaluer l'influence des ions carbonates sur les données spectrales lors de la quantification du Fl1. Tout d'abord une solution à 50 ppm de Fl1 est préparée par dilution de la solution TEST2. Avec cette nouvelle solution, 4 échantillons sont préparés à 0, 1000, 4000 et 7000 ppm de carbonate de calcium.

1 mL de chacun de ces échantillons sont placés dans des flacons de 10 mL en présence de 9 mL de la solution de l'exemple 2 à 100 ppm de sel d'europium (Eu-LD10020). Après une heure, 2 mL de ces nouvelles solutions sont analysés selon les conditions expérimentales définies ci-dessus. Les spectres obtenus sont présentés en Figure 9. Les spectres d'émissions ne montrent pas d'effets attribuables aux carbonates sur la mesure malgré l'apparition d'un précipité pour les fortes concentrations en carbonates.

## Revendications

1. Méthode de détection d'additifs dans un fluide aqueux comprenant:
a. le mélange d'une solution révélatrice comprenant un cation lanthanide et un agent chélateur des lanthanides, avec un échantillon du fluide aqueux à analyser comprenant éventuellement au moins un additif, dans des conditions permettant la complexation du lanthanide par l'agent chélateur et par l'additif éventuellement présent,
b. La détection et, le cas échéant, la quantification, de la variation de fluorescence liée à la présence éventuelle de l'additif dans le fluide aqueux par fluorescence en temps résolu,
**caractérisée en ce que** l'agent chélateur est choisi parmi la diaminopyridine, l'imidazoline, le polyanhydride maléique hydolysé, l'acide oxalique, l'acetylacetonate, le thiodiacétate, l'acide nitrilotriacétique (NTA), ou leurs dérivés.

2. Méthode selon la revendication 1, **caractérisée en ce que** la solution révélatrice comprend en outre au moins 1g/L d'ions chlorure, et de préférence une concentration en ions chlorure comprise entre 10 et 50g/L.

3. Méthode selon l'une des revendications 1 à 2, **caractérisée en ce que** la solution révélatrice comprend en outre au moins 1g/L d'un composé chimique utilisé dans la fabrication de solution tampon, par l'acide 4-(2-hydroxyéthyl)-1-pipérazine éthane sulfonique (HEPES).

4. Méthode selon l'une des revendications 1 à 3, **caractérisée en ce que** le rapport de concentration entre l'agent chélateur et le lanthanide est compris entre 1 :10 et 10 :1, par exemple entre 1 :3 et 3 :1.

5. Méthode selon l'une des revendications 1 à 4, **caractérisée en ce que** le lanthanide est choisi parmi : Pr, Nd, Sm, Eu, Tb, Dy, Ho, Er, Tm et Yb, ainsi que leurs mélanges, de préférence Eu.

6. Méthode selon l'une des revendications 1 à 5, **caractérisée en ce que** ledit additif à détecter est un inhibiteur de dépôts minéraux ou de corrosion.

7. Méthode selon la revendication 6, **caractérisée en ce que** l'inhibiteur de dépôts minéraux est choisi parmi :
- les polyphosphates,
- les organophosphonates,
- les polyacides carboxyliques,
- les polymères à fonction acide sulfonique, en particulier les copolymères d'acide styrène sulfonique et d'acide maléique,
- l'acide polyphosphinocarboxylique (PPCA) éventuellement sulfonaté,
- la polyéthylèneimine (PEI),
- les polymères siliconés fonctionnalisés par des groupements amines, et
- les copolymères à base d'ammoniums quaternaires.

8. Méthode selon la revendication 6, **caractérisée en ce que** l'inhibiteur de corrosion est choisi parmi la cyclohexylamine, l'hexylamine, la morpholine, l'octadécylamine, le diéthylaminoéthanol, les imidazolines et les bétaïnes.

9. Méthode selon l'une des revendications 1 à 8, **caractérisée en ce que** le délai d'intégration entre l'excitation de l'échantillon à analyser et la mesure du signal émis est compris entre 0,001 et 10 ms, de préférence entre 0,01 et 5 ms, plus préférentiellement entre 0,1 et 3 ms.

10. Solution révélatrice pour la détection d'additifs et notamment d'inhibiteurs de dépôts minéraux ou de corrosion, comprenant :
(i) un cation lanthanide, par exemple Eu3+,
(ii) un agent chélateur des lanthanides, choisi parmi la diaminopyridine, l'imidazoline, le polyanhydride maléique hydolysé, l'acide oxalique, l'acétylacétonate, le thiodiacétate, ou leurs dérivés, l'acide nitrilotriacétique (NTA) ;
(iii) des ions chlorure à plus de 1 g/L ;
(iv) un composé chimique utilisé dans la fabrication de solution tampon à plus de 1g/L, par exemple l'HEPES ;
le rapport de concentration entre l'agent chélateur et le lanthanide étant compris entre 1 :10 et 10 :1, de préférence entre 1 :3 et 3 :1.

11. Solution révélatrice selon la revendication 10, comprenant entre 10 et 1000ppm de EuCl₃.6H₂0, 10 et 5000ppm d'imidazoline et entre 5 et 50g/L de NaCl, à pH compris entre 4 et 8.

12. Solution révélatrice selon la revendication 10, comprenant entre 1 et 100 ppm de EuCl₃.6H₂0, 2 et 200ppm de polyanhydride maléique hydrolysé et entre 5 et 50g/L de NaCl, à pH compris entre 4 et 8.

13. Utilisation d'une solution révélatrice selon l'une des revendications 10 -12 pour détecter et, le cas échéant, quantifier, un additif dans un fluide aqueux, par exemple un inhibiteur de dépôts minéraux ou de corrosion dans un fluide issu d'un puits de production de pétrole ou de gaz.

## Patentansprüche

1. Verfahren zum Nachweis von Additiven in einer wässrigen Flüssigkeit, beinhaltend:
a. Mischen einer Nachweislösung, die ein Lanthanidkation und einen Lanthanid-Chelatbildner beinhaltet, mit einer Probe der zu analysierenden wässrigen Flüssigkeit, die gegebenenfalls mindestens ein Additiv beinhaltet, unter Bedingungen, die eine Komplexierung des Lanthanids durch den Chelatbildner und durch das gegebenenfalls vorhandene Additiv ermöglichen,
b. Nachweis und gegebenenfalls Quantifizierung der Änderung der Fluoreszenz im Zusammenhang mit der möglichen Anwesenheit des Additivs in der wässrigen Flüssigkeit durch Fluoreszenz in Abhängigkeit von der Zeit,
**dadurch gekennzeichnet, dass** der Chelatbildner ausgewählt ist aus Diaminopyridin, Imidazolin, hydolysiertem Maleinsäurepolyanhydrid, Oxalsäure, Acetylacetonat, Thiodiacetat, Nitrilotriessigsäure (NTA) oder Derivaten davon.

2. Verfahren nach Anspruch 1, **dadurch gekennzeichnet, daß** die Nachweislösung ferner mindestens 1 g/l Chloridionen und vorzugsweise eine Chloridionenkonzentration zwischen 10 und 50 g/l enthält.

3. Verfahren nach einem der Ansprüche 1 bis 2, **dadurch gekennzeichnet, daß** die Nachweislösung ferner mindestens 1g/L einer chemischen Verbindung enthält, die bei der Herstellung einer Pufferlösung durch 4-(2-Hydroxyethyl)-1-piperazinethansulfonsäure (HEPES) verwendet wird.

4. Verfahren nach einem der Ansprüche 1 bis 3, **dadurch gekennzeichnet, daß** das Konzentrationsverhältnis zwischen Chelatbildner und Lanthanid zwischen 1:10 und 10:1, z.B. zwischen 1:3 und 3:1, liegt.

5. Verfahren nach einem der Ansprüche 1 bis 4, **dadurch gekennzeichnet, daß** das Lanthanid ausgewählt ist aus : Pr, Nd, Sm, Eu, Tb, Dy, Ho, Er, Tm und Yb sowie Mischungen davon, vorzugsweise Eu.

6. Verfahren nach einem der Ansprüche 1 bis 5, **dadurch gekennzeichnet, dass** der nachzuweisende Additiv ein Inhibitor von Mineralablagerungen oder Korrosion ist.

7. Verfahren nach Anspruch 6, **dadurch gekennzeichnet, dass** der Mineralablagerungshemmer ausgewählt ist aus :
- Polyphosphate,
- Organophosphonate,
- Polycarbonsäuren,
- Sulfonsäure-funktionelle Polymere, insbesondere Copolymere aus Styrolsulfonsäure und Maleinsäure,
- Polyphosphinocarbonsäure (PPCA), gegebenenfalls sulfoniert,
- Polyethylenimin (PEI),
- aminfunktionalisierte Silikonpolymere und
- Copolymere auf der Basis von quaternären Ammoniumverbindungen.

8. Verfahren nach Anspruch 6, **dadurch gekennzeichnet, dass** der Korrosionsinhibitor ausgewählt ist aus Cyclohexylamin, Hexylamin, Morpholin, Octadecylamin, Diethylaminoethanol, Imidazolinen und Betainen.

9. Verfahren nach einem der Ansprüche 1 bis 8, **dadurch gekennzeichnet, dass** die Integrationszeit zwischen der Anregung der zu analysierenden Probe und der Messung des emittierten Signals zwischen 0,001 und 10 ms, vorzugsweise zwischen 0,01 und 5 ms, besonders bevorzugt zwischen 0,1 und 3 ms, liegt.

10. Nachweislösung zum Nachweis von Additiven, insbesondere Inhibitoren von Mineralablagerungen oder Korrosion, bestehend aus :
(i) ein Lanthanoid-Kation, z.B. Eu³⁺,
(ii) ein Lanthanid-Chelatbildner, ausgewählt aus Diaminopyridin, Imidazolin, hydolysiertem Malein-Polyanhydrid, Oxalsäure, Acetylacetonat, Thiodiacetat oder Derivaten davon, Nitrilotriessigsäure (NTA) ;
(iii) Chloridionen über 1 g/L ;
(iv) eine chemische Verbindung, die bei der Herstellung von Pufferlösungen über 1g/L verwendet wird, z.B. HEPES ;
wobei das Konzentrationsverhältnis zwischen dem Chelatbildner und dem Lanthanid zwischen 1:10 und 10:1, vorzugsweise zwischen 1:3 und 3:1, liegt.

11. Nachweislösung nach Anspruch 10, die zwischen 10 und 1000ppm EuCl₃·6 H₂0, 10 und 5000ppm Imidazolin und zwischen 5 und 50g/L NaCl enthält, bei einem pH-Wert zwischen 4 und 8.

12. Nachweislösung nach Anspruch 10, die zwischen 1 und 100 ppm EuCl₃·6 H₂0, 2 und 200 ppm hydrolysiertes Polymaleinsäureanhydrid und zwischen 5 und 50 g/l NaCl enthält, bei einem pH-Wert zwischen 4 und 8.

13. Verwendung einer Nachweislösung nach einem der Ansprüche 10 bis 12 zum Nachweis und gegebenenfalls zur Quantifizierung eines Additivs in einer wäßrigen Flüssigkeit, beispielsweise eines Inhibitors von Mineralablagerungen oder Korrosion in einer Flüssigkeit aus einer Öl- oder Gasförderbohrung.

## Claims

1. Method for detecting additives in an aqueous fluid, comprising:
a. mixing a detecting solution comprising a lanthanide cation and a lanthanide chelating agent, with a sample of the aqueous fluid to be analysed optionally comprising at least one additive, under conditions allowing complexing of the lanthanide by the chelating agent and by the additive that is optionally present,
b. detecting and, if appropriate, quantifying the variation in fluorescence associated with the possible presence of the additive in the aqueous fluid by time-resolved fluorescence,
**characterized in that** the chelating agent is selected from diaminopyridine, imidazoline, hydrolysed polymaleic anhydride, oxalic acid, acetylacetonate, thiodiacetate, nitrilotriacetic acid (NTA), or derivatives thereof.

2. Method according to claim 1, **characterized in that** the detecting solution further comprises at least 1 g/L of chloride ions, and preferably a concentration of chloride ions comprised between 10 and 50 g/L.

3. Method according to one of claims 1 to 2, **characterized in that** the detecting solution further comprises at least 1 g/L of a chemical compound used in the production of buffer solution, by 4-(2-hydroxyethyl)-1-piperazine-ethanesulphonic acid (HEPES).

4. Method according to one of claims 1 to 3, **characterized in that** the concentration ratio of the chelating agent to the lanthanide is comprised between 1:10 and 10:1, for example between 1:3 and 3:1.

5. Method according to one of claims 1 to 4, **characterized in that** the lanthanide is selected from: Pr, Nd, Sm, Eu, Tb, Dy, Ho, Er, Tm and Yb, and mixtures thereof, preferably Eu.

6. Method according to one of claims 1 to 5, **characterized in that** said additive to be detected is an inhibitor of mineral deposits or of corrosion.

7. Method according to claim 6, **characterized in that** the inhibitor of mineral deposits is selected from:
- polyphosphates,
- organophosphonates,
- polycarboxylic acids,
- polymers with a sulphonic acid function, in particular the copolymers of styrenesulphonic acid and maleic acid,
- polyphosphinocarboxylic acid (PPCA), optionally sulphonated,
- polyethyleneimine (PEI),
- silicone polymers functionalized with amine groups, and
- copolymers based on quaternary ammonium.

8. Method according to claim 6, **characterized in that** the corrosion inhibitor is selected from cyclohexylamine, hexylamine, morpholine, octadecylamine, diethylaminoethanol, the imidazolines and the betaines.

9. Method according to one of claims 1 to 8, **characterized in that** the integration delay between excitation of the sample to be analysed and measurement of the signal emitted is between 0.001 and 10 ms, preferably comprised between 0.01 and 5 ms, more preferably between 0.1 and 3 ms.

10. Detecting solution for detecting additives and in particular inhibitors of mineral deposits or of corrosion, comprising:
(i) a lanthanide cation, for example Eu3+,
(ii) a lanthanide chelating agent, for example selected from diaminopyridine, imidazoline, hydrolysed polymaleic anhydride, oxalic acid, acetylacetonate, thiodiacetate, or derivatives thereof, nitrilotriacetic acid (NTA);
(iii) chloride ions at more than 1 g/L;
(iv) a chemical compound used in the production of buffer solution at more than 1 g/L, for example HEPES;
the concentration ratio of the chelating agent to the lanthanide being comprised between 1:10 and 10:1, preferably between 1:3 and 3:1.

11. Detecting solution according to claim 10, comprising between 10 and 1000 ppm of EuCl₃.6H₂O, 10 and 5000 ppm of imidazoline and between 5 and 50 g/L of NaCl, at a pH comprised between 4 and 8.

12. Detecting solution according to claim 10, comprising between 1 and 100 ppm of EuCl₃.6H₂O, 2 and 200 ppm of hydrolysed poly(maleic anhydride) and between 5 and 50 g/L of NaCl, at a pH comprised between 4 and 8.

13. Use of a detecting solution according to one of claims 10 to 12 for detecting and, if appropriate, quantifying an additive in an aqueous fluid, for example an inhibitor of mineral deposits or of corrosion in a fluid taken from a producing well for oil or gas.
